# EUROPEAN PATENT SPECIFICATION

(11) **EP 2 768 426 B1**
(45) Date of publication and mention of the grant of the patent: **23.03.2016**
(21) Application number: 12780974.7
(22) Date of filing: 11.10.2012
(51) Int. Cl.: A61F 2/01

(54) **EMBOLIC PROTECTION DEVICES**
EMBOLIESCHUTZVORRICHTUNGEN
DISPOSITIFS DE PROTECTION EMBOLIQUE

(30) Priority: 17.10.2011 US 201161548130 P; 16.04.2012 US 201213448277
(43) Date of publication of application: 27.08.2014
(73) Proprietor: W.L. Gore & Associates, Inc., Newark, DE 19711 (US)
(72) Inventor: BRUCHMAN, William, C., Newark, DE 19714-9206 (US); CULLY, Edward, H., Newark, DE 19714-9206 (US); HARTMAN, Cody, L., Newark, DE 19714-9206 (US); LOVEKAMP, Joshua, J., Newark, DE 19714-9206 (US); TRAPP, Benjamin, M., Newark, DE 19714-9206 (US)
(74) Representative: Wilson, Gary
(86) International application number: PCT/US2012/059769
(87) International publication number: WO 2013/059069

(56) References cited:
- WO-A1-99/32050
- WO-A2-99/16382
- US-A1- 2010 168 785
- US-A1- 2010 305 604
- US-A1- 2011 137 399

## Description

### CROSS-REFERENCE TO RELATED APPLICATION

### BACKGROUND

### Field

The disclosure relates to embolic protection during endovascular procedures. as described in claim 1. Where in the following the word invention is used and/or features are presented as optional this should be interpreted in such way that protection is sought for the invention as claimed.

### Discussion of the Related Art

Circulation of embolic debris can cause mild to extreme cardiovascular complications, leading to stroke and even death. While the sloughing of embolic debris from the internal walls of the vasculature can be unpredictable, various endovascular procedures may inadvertently be associated with the release of embolic debris.

Typical embolic protection devices used in connection with such endovascular procedures either capture embolic debris and must be removed with embolic debris captured therein, or merely redirect (i.e., do not remove) embolic debris to vasculature where it presents a lower risk (e.g., away from the carotid arteries to the peripheral vasculature, capillaries and tissue bed).

Removing an embolic protection device with embolic debris captured therein necessitates a relatively large crossing profile. There is also a risk of unintentionally releasing a large volume of embolic debris into the vasculature during the removal process, for example, in the case of operator error or if a device malfunction occurs. Merely redirecting embolic debris on the other hand, does not eliminate, but only delays or potentially re-focuses the risk of cardiovascular complications to another anatomical region. There is thus a need for improved embolic protection devices, systems and methods. The present disclosure addresses this need.

US2011137399 (A1) discloses an apparatus that is useful in an embolization procedure and enables substantially unrestricted forward flow of blood in a vessel and reduces or stops reflux (regurgitation or backward flow) of embolization agents which are introduced into the blood. The deployable apparatus includes a delivery catheter having a valve fixedly coupled to the distal end thereof.

### SUMMARY

Embolic protection devices, systems and methods are provided. An embodiment of an embolic protection device is provided comprising an elongate element having a deflector at its distal end. The embolic protection device is generally configured to redirect and/or funnel embolic debris and permit at least a portion of the blood, no longer containing the redirected and/or funneled embolic debris, to perfuse the surrounding vasculature and/or tissue.

### BRIEF DESCRIPTION OF THE DRAWINGS

The accompanying drawings are included to provide a further understanding of the disclosure and are incorporated in and constitute a part of this specification, illustrate embodiments of the disclosure, and together with the description serve to explain the principles of the disclosure.
Figure 1A illustrates an embolic protection device in accordance with an embodiment;
Figure 1B illustrates a close-up of the deflector of the embolic protection device illustrated in Figure 1 A;
Figure 2 illustrates an embolic protection device deployed in the aortic arch in accordance with an embodiment;
Figure 3A illustrates an embolic protection device having a permeable deflector deployed in the aortic arch in accordance with an embodiment;
Figure 3B illustrates an embolic protection device having a deflector with a variable pore size deployed in the aortic arch in accordance with an embodiment;
Figure 4A illustrates an embolic protection device comprising an elongate element having permeable windows deployed in the aortic arch in accordance with an embodiment;
Figure 4B illustrates an embolic protection device comprising an elongate element having a permeable window deployed in a carotid artery in accordance with an embodiment;
Figure 5 illustrates an embolic protection system in accordance with an embodiment;
Figures 6A-6C illustrate yet another embolic protection device in connection with a multi-stage deployment in accordance with an embodiment;
Figure 7A illustrates an embolic protection device comprising a deflector configured to be deconstructed in situ in accordance with an embodiment; and
Figure 7B illustrates an embolic protection device comprising a deflector after having been deconstructed in situ in accordance with an embodiment.

### DETAILED DESCRIPTION OF THE ILLUSTRATED EMBODIMENTS

Persons skilled in the art will readily appreciate that various aspects of the present disclosure may be realized by any number of methods and apparatuses configured to perform the intended functions. Stated differently, other methods and apparatuses may be incorporated herein to perform the intended functions. It should also be noted that the accompanying drawing figures referred to herein are not all drawn to scale, but may be exaggerated to illustrate various aspects of the present disclosure, and in that regard, the drawing figures should not be construed as limiting. Finally, although the present disclosure may be described in connection with various principles and beliefs, the present disclosure should not be bound by theory.

The terms "downstream" or "antegrade" and "upstream" or "retrograde," when used herein in relation to the patient's vasculature, refer to the direction of blood flow and the direction opposite that of blood flow, respectively. In the arterial system, "downstream" or "antegrade" refers to the direction further from the heart, while "upstream" or "retrograde" refers to the direction closer to the heart. In this regard, "retrograde vascular delivery" as used herein means delivered to a treatment site in the direction opposite that of blood flow. The terms "proximal" and "distal," when used herein in relation to a device or device component, refer respectively, to directions closer to the device's hub or operator (and farther away from the device's tip) and closer to the device's tip (and farther away from the device's hub or operator). Since the present disclosure is not limited to peripheral or central approaches, the device should not be narrowly construed when using the terms proximal or distal since device features can be slightly altered relative to the anatomical features and the device position relative thereto.

According to one aspect of an embodiment, protection against embolic debris is provided. As used herein, "embolic debris" means biologic or non-biologic elements, the presence of which in the vasculature presents an embolic risk (including, but not limited to plaque, emboli, etc.).

According to an embodiment, an embolic protection device is provided comprising an elongate element having a deflector at or near its distal end. The embolic protection device is generally configured to redirect and/or funnel embolic debris and permit at least a portion of the blood, no longer containing emboli of a clinically relevant size, to perfuse the surrounding vasculature and/or tissue.

As used herein, an elongate element is generally any structure configured to receive embolic debris redirected and/or funneled from the deflector. An elongate element can be further configured to provide a working lumen through which embolic debris can be aspirated and/or through which one or more secondary endovascular devices (e.g., pig tail catheters, balloon valvuloplasty catheters, heart valve delivery catheters, balloon catheters used in connection with carotid artery stenting flow reversal procedure, thromectomy, atherectomy and embolectomy devices, tools, etc.) and/or prostheses (e.g., tri-leaflet valve, etc.) can be delivered to a treatment site. While delivery of one or more secondary endovascular devices and/or prostheses can occur subsequent to delivery of an elongate element itself to a treatment site, an elongate element can be delivered together with one or more secondary endovascular devices and/or prostheses. By way of non-limiting example, an elongate element can be delivered together with a balloon catheter extending from a distal opening thereof.

An example of an embodiment of an elongate element comprises a proximal opening and a distal opening and comprises at least one lumen extending therethrough. In this regard, an elongate element can comprise a separate lumen, e.g., for delivering a blood contrast agent, or for enclosing a deployment or release line for a sleeve, sheath, sock or other constraining mechanism, a steering line, or a structural support element. An elongate element can comprise an introducer or a catheter, among other components.

As used herein, a deflector is generally any structure configured to redirect and/or funnel embolic debris into the elongate element. A deflector can be further configured to not capture or retain embolic debris, but rather, to redirect and/or funnel embolic debris. A deflector can comprise a proximal opening, a distal opening, and at least one lumen extending therethrough. In some embodiments, at least a portion of the deflector has a cross-section greater than that of the distal opening of the elongate element. In some embodiments, at least a portion of the deflector has a cross-section substantially equal to that of the vessel or lumen where the deflector is deployed. In other embodiments, the deflector has a maximum cross-section less than that of the vessel or lumen where the deflector is deployed. A deflector can have any number of configurations, for example, a generally frustoconical, cylindrical and/or trumpet shape. In various embodiments, a deflector is circumferential and comprises a lumen extending therethrough. In addition, a deflector can be selectively diametrically adjustable.

In some embodiments, a deflector having a generally frustoconical shape can be particularly advantageous in applications where contact with the inner wall of the vessel or lumen or one or more branch vessels or lumens can cause undesirable side effects such as the disruption and/or release of additional embolic debris. A deflector having a generally frustoconical shape can also be advantageous in applications where the vessel or lumen to where the deflector is deployed is particularly tortuous and gradual turning is advised for the safe and/or efficient delivery of secondary endovascular devices and/or prostheses.

In various embodiments, a deflector can have a radially collapsed delivery configuration and a radially expanded redirecting and/or funneling configuration. A deflector can further be configured to be self-expanding or have one or more self-expanding elements. In the alternative, a deflector can be configured to assume its radially expanded funneling configuration by employing a balloon catheter system.

In embodiments, the deflector and/or a portion thereof is restrained or otherwise covered in a radially collapsed delivery configuration by a releasable or removable cover such as a sleeve, sheath, sock or other constraining mechanism. In other embodiments, the deflector and/or a portion thereof is restrained or otherwise covered in a radially collapsed delivery configuration by a surrounding tubular element until it is deployed therefrom by relative axial movement of the deflector and the surrounding tubular element. Deployment of the deflector can occur proximal to distal, distal to proximal, ends inward, center outward, etc.

Additional deployment systems are contemplated, for example, those taught in U.S. Pat. Nos. 6,315,792 and 6,224,627, both by Armstrong et al., and both of which are hereby incorporated by reference in their entireties for all purposes. By way of non-limiting example, a deployment system can comprise a cover for a self-expanding device that is both effective at maintaining the self-expanding device in a constrained initial orientation and is easily removable during the deployment procedure. The deployment system can comprise a warp knit (also known as a "knit-braid") of two or more interlocking strands of thread that forms a relatively tight cover (or "encasing") around the self-expanding device. Each of the threads covers only a portion of the outer circumference of a radially compressed device. By imparting a break in one filament of the knit-braid at one end of the cover, the cover can be removed in its entirety through simple application of tension in any direction to a multi-filament "rip cord." The rip cord can be contiguous with the cover, which allows the cover to be removed in its entirety when subjected to tension while the self-expanding device expands in place. This construction has many advantages over previous deployment systems, including allowing removal of the cover along a single vector without the deployment rip cord undergoing the windshield-wiper effect.

In various embodiments, to be discussed in greater detail below, a distal end of a deflector is maintained separately in a radially collapsed configuration even after deployment of a proximal portion of the deflector. In such embodiments, the deflector can be configured to allow perfusion therethrough even while the distal end of a deflector is in a radially collapsed configuration.

Other embodiments can be steerable. For example, the elongate element and/or the deflector can be housed in a sleeve, sheath, sock or other constraining mechanism. Such a constraining mechanism (and/or the elongate element and/or the deflector itself) can have a deployment line which, if locked (e.g., pin, wire or other), acts as a tension line to cause bending of the elongate element and/or the deflector. Steerable embodiments can be particularly beneficial in treating the aortic arch or other tortuous vasculature. Steerable embodiments can be particularly useful in preventing the deflector from compressing against the outside of a bend in a tortuous anatomy. Such steerable embodiments can also be useful in creating an exit from the elongate element that is substantially aligned with the axis of the vessel and the axis of the deflector mechanism.

In certain embodiments, a distal opening of the elongate element is coupled with a proximal end of the deflector. In such embodiments, the coupling can be permanent or temporary. In embodiments, the deflector is slideably deliverable (outside or within the elongate element) from the proximal opening to the distal opening of the elongate element. In other embodiments, the elongate element and the deflector are integral, for example, one projecting from the end of the other and/or formed on a common mandrel from one or more common materials.

Elongate elements and deflectors can comprise materials that are substantially permeable, semi-permeable or impermeable depending on the particular application. Persons skilled in the art will appreciate that permeability can arise from either or both of material properties (e.g., node and fibril configuration of an expanded fluoropolymer, material porosity, average pore size, etc.), or a woven, knitted or lattice configuration, perforations, laser cut holes, to name a few. In general, and as described supra, the permeability is selected to redirect embolic debris of a predetermined size. For example, elongate elements and deflectors can be configured to redirect embolic debris yet also permit perfusion therethrough and/or delivery of a therapeutic agent to the surrounding vasculature and/or tissue. Other elongate elements and deflectors can be configured to not permit perfusion therethrough.

Moreover, an elongate element or deflector can have a variable pore size, for example, from a proximal end to a distal end, or at one or more discrete locations (e.g., one or more permeable windows). By way of non-limiting example, a distal portion can have a smaller pore size to not let smaller embolic debris enter into the great vessels, whereas a proximal portion can have a larger pore size beyond a point where entry of embolic debris into the great vessels is not a concern.

Permeable windows can be located anywhere along or about an elongate element or deflector and can comprise various suitable dimensions (including, but not limited to circular, ovoidal, elongated, spiral, random, etc.). In embodiments, an elongate element or deflector can comprise at least one window having a greater porosity than an adjacent portion of the elongate element or deflector.

In various embodiments, one or more permeable windows can be used to reduce or eliminate the likelihood of a stagnant column of blood within an elongate element or a deflector. Trapped embolic debris which are rendered stagnant can decrease porosity and increase the pressure gradient across the elongate element or the deflector. In turn, embolic debris which are rendered stagnant can, over time, build up and decrease filtering efficiency, which may be especially problematic in a challenging environment such as the aorta which has such great output. An elongate element having one or more permeable windows on the other hand, can allow blood and redirected embolic debris to migrate down and into its lumen, where it's collection will have no detrimental effect on filtering efficiency, and where it's removal by aspiration can be accomplished by the operator.

A permeable window can comprise a one-way flap or valve to permit perfusion therethrough yet prevent blood from entering, for example, during an aspiration procedure. An embodiment of a one-way valve can comprise a biocompatible material (e.g., a fluoropolymer) having one or more slits and being biased to open in one direction. An embodiment of a one-way flap can comprise a biocompatible material (e.g., a fluoropolymer) with a support frame. Embodiments of a one-way flap or valve can be positioned on the outer and/or inner surface of a permeable window in such a manner to substantially cover the window.

In like manner, elongate elements and deflectors can comprise one or more one-way flaps or valves in a lumen thereof to prevent embolic debris from exiting once redirected and/or funneled by the embolic protection device.

In various embodiments, at least one of the elongate element and the deflector in an embolic protection device is configured to redirect embolic debris (i.e., not be permeable to embolic debris) greater than or equal to about 100µm, about 80µm, and/or about 40µm (as measured lengthwise across its longest dimension). Such redirection may result from the respective element(s) having an average pore size less than or equal to about 100µm, about 80µm, and/or about 40µm (as measured lengthwise across its longest dimension). As discussed supra, redirection may vary along or about an elongate element or deflector. By way of non-limiting example, a deflector distal portion can have an average pore size of about 40µm to not let smaller embolic debris enter into the great vessels, whereas a deflector proximal portion can have an average pore size of about 100µm beyond a point where entry of embolic debris into the great vessels is not a concern.

In embodiments, at least one of the elongate element and the deflector in an embolic protection device is configured to permit perfusion and/or delivery of a therapeutic agent to the surrounding vasculature and/or tissue.

Elongate elements and deflectors can comprise various materials including, but not limited to polymers, such as fluoropolymers like an expanded polytetrafluoroethylene ("ePTFE"), expanded PTFE, expanded modified PTFE, expanded copolymers of PTFE, nylons, polycarbonates, polyethylenes, polypropylenes and the like.

Elongate elements and deflectors can comprise one or more structural support elements, for example, braids, meshes, lattices, wires or ring or helical stent elements, any the foregoing either laser cut from a tube or formed separately. Structural support elements can comprise a shape-memory material, such as nitinol. In other embodiments, however, structural support elements can be comprised of other materials, self-expandable or otherwise expandable (e.g., with a fluid-filled balloon), such as various metals (e.g., stainless steel), alloys and polymers.

In embodiments, a deflector can be deconstructed in situ by applying tension to a structural support element, for example, as taught in U.S. Ser. No. 13/324,187 and U.S. Pub. Nos. 2010/0069916, 2009/0259294 and 2005/0131515, all by Cully et al.,

By way of non-limiting example, the deflector can include a helically-wound structural support element provided with a covering of deflector material. The deflector can be removable by gripping an end of the helically-wound structural support element with a retrieval device and applying tension to the structural support element in the direction in which it is intended to be withdrawn from the site of implantation. The use of such a retrieval device allows the deflector to be removed remotely, such as via a catheter inserted into the body at a different location from the implantation site. The design of the deflector is such that the structural support element can be extended axially while the adjacent portion of the deflector material separates from the windings of the structural support element. The axial extension of the structural support element, with portions of the deflector material still joined to the structural support element, allows the device to be "unraveled" (or "unwound") and removed through a catheter of diameter adequately small to be inserted into the body cavity that contained the deflector. It can be removed atraumatically, without incurring significant trauma to the body conduit in which it had been deployed.

More generally, deconstruction can occur proximal to distal, distal to proximal, ends inward, center outward, etc. Moreover, deconstruction can comprise removal of a structural support element from a deflector material or removal together with a deflector material. Deconstruction can be facilitated by a perforated or weakened deflector material, bioabsorbable glue, etc. In-situ deconstruction can negate the need for another over-sheath to capture the deflector, thus reducing crossing profile. In embodiments, a deflector can be recaptured with an over-sheath.

Elongate elements and deflectors can comprise a therapeutic agent, for example, be coated or imbibed with a therapeutic agent, whether dry, gel or liquid. Examples of therapeutic agents comprise antiproliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP)IIbIIIa inhibitors and vitronectin receptor antagonists; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), trazenes-dacarbazinine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine{cladribine}); platinum coordination complexes (cisplatin, carboplatin), procarbazine, hydroxyurea, mitotane, aminoglutethimide; hormones (i.e. estrogen); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fludrocortisone, prednisone, prednisolone, 6α-methylprednisolone, triamcinolone, betamethasone, and dexamethasone), non-steroidal agents (salicylic acid derivatives i.e. aspirin; para-aminophenol derivatives i.e. acetominophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF) platelet derived growth factor (PDGF), erythropoetin; angiotensin receptor blocker; nitric oxide donors; anti-sense oligionucleotides and combinations thereof; cell cycle inhibitors, mTOR inhibitors, growth factor signal transduction kinase inhibitors, chemical compound, biological molecule, nucleic acids such as DNA and RNA, amino acids, peptide, protein or combinations thereof.

Any portion of the elongate element or the deflector can comprise a radio-opaque or echogenic element that enhances imaging or detection during and/or following delivery or deployment. For instance, radio-opaque markers or bands may be incorporated at a proximal end of a deflector, a distal end of a deflector, and/or a location on or about a deflector where an average pore size changes or transitions. Radio-opaque markers or bands can be comprised of one or more of tungsten, gold, platinum and the like.

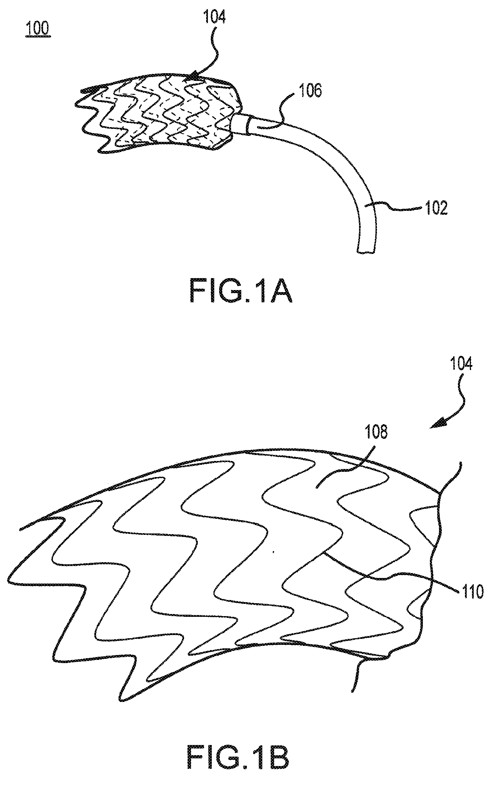

By way of non-limiting examples in the aortic arch, and with reference now to the figures, FIG. 1A illustrates an embolic protection device 100 comprising an elongate element 102 having a cylindrically shaped deflector 104 at the distal opening 106 of elongate element 102. FIG. 1B illustrates a close-up of deflector 104. In this embodiment, deflector 104 comprises a helically wound structural support element 110 and deflector material 108. The deflector material 108 comprises perforated ePTFE material. Deflector material 108 is configured to redirect embolic debris yet also to permit perfusion to the surrounding vasculature and/or tissue.

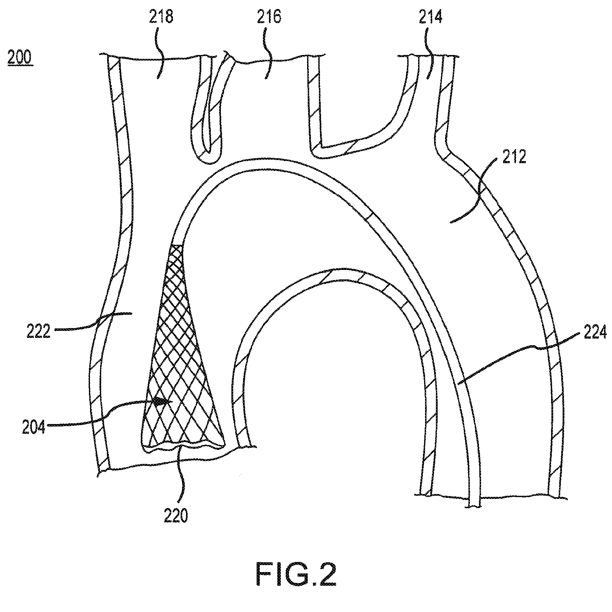

FIG. 2 illustrates an embolic protection device 200 deployed in aortic arch 212, wherein a deflector 204 of embolic protection device 200 is deployed upstream from left subclavian artery 214, left common carotid artery 216 and brachiocephalic artery 218. In this illustrated embodiment, deflector 204 of embolic protection device 200 has a generally frustoconical shape. In this embodiment, a distal end 220 of deflector 204 has a cross-sectional surface area less than that of ascending aorta 222 where deflector 204 is deployed, thereby allowing perfusion to left subclavian artery 214, left common carotid artery 216 and brachiocephalic artery 218, and also allowing perfusion through aortic arch 212 to descending aorta 224 and downstream vasculature. FIG. 2 illustrates a steerable embodiment wherein the direction of the elongate element 224 is controlled remotely by the operator thereby reducing the dependence on the vessel wall for alignment. This is shown to result in no compression of the deflector 204 against the outside curvature of the vessel and to result in an axis of the vessel, the elongate element 224, and the deflector 204 being substantially aligned.

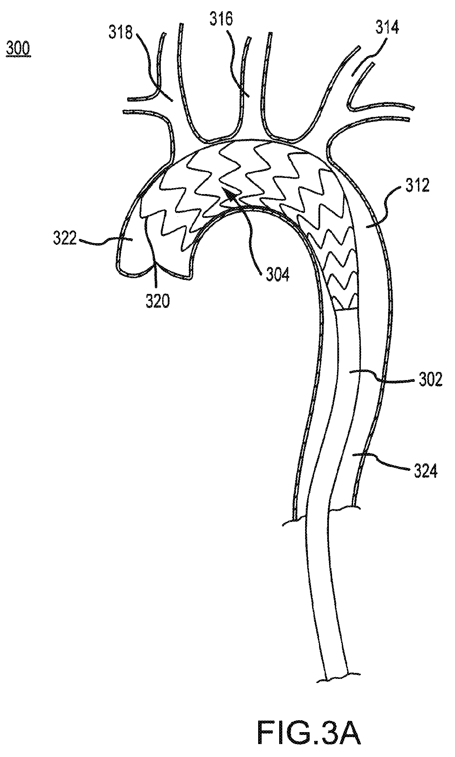

With reference to FIG. 3A, an embolic protection device 300 is illustrated deployed in aortic arch 312 having a blood-permeable deflector 304 and a blood-impermeable elongate element 302. In this embodiment, a distal end 320 of deflector 304 has a cross-sectional surface area substantially equal to that of ascending aorta 322 where deflector 304 is deployed, thereby directing all blood flow into deflector 304. Because deflector 304 is blood-permeable, perfusion to left subclavian artery 314, left common carotid artery 316 and brachiocephalic artery 318, and perfusion through aortic arch 312 to descending aorta 324 and downstream vasculature are made possible. In this embodiment, due to the combined frustoconical-cylindrical shape of deflector 304, contact is made with each of the openings to left subclavian artery 314, left common carotid artery 316 and brachiocephalic artery 318.

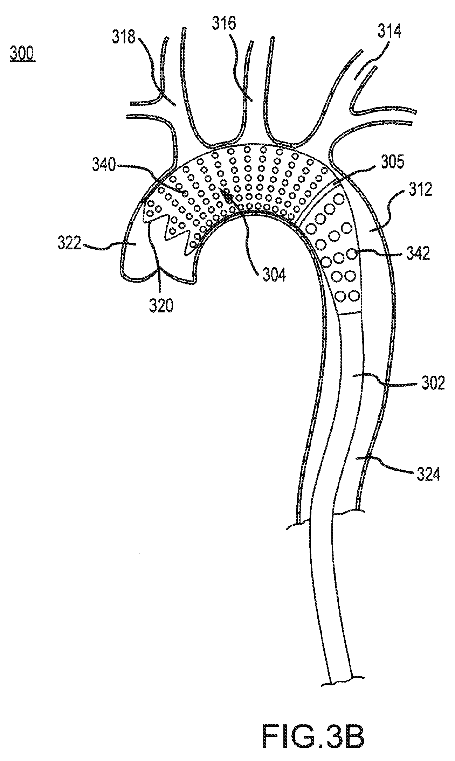

FIG. 3B is similar to FIG, 3A, except deflector 304 is illustrated with deflector material having a variable pore size. More specifically, a distal portion of deflector 304 comprises a plurality of first pores 340 having a smaller average pore size to not let smaller embolic debris enter into left subclavian artery 314, left common carotid artery 316 or brachiocephalic artery 318. A proximal portion of deflector 304 comprises a plurality of second pores 342 having a larger average pore size beyond left subclavian artery 314 where entry of embolic debris into the great vessels is not a concern. In addition, the deflector 304 includes a radio-opaque band 305 between the proximal and distal portions to facilitate proper placement of deflector 304 relative to the respective vasculature.

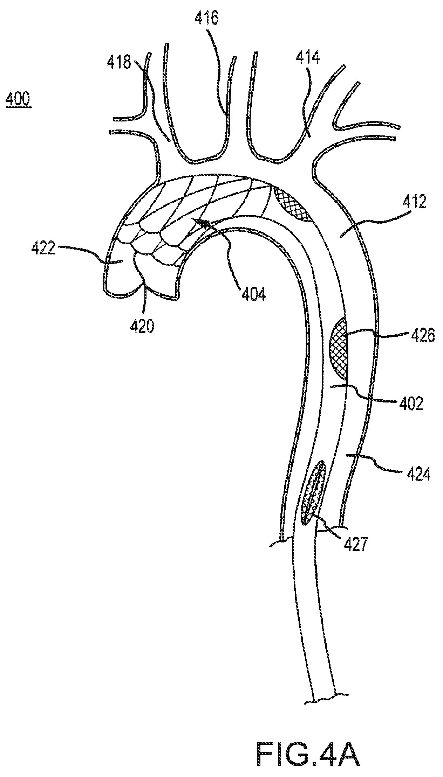

With reference now to FIG. 4A, an embolic protection device 400 is illustrated deployed in aortic arch 412 comprising a blood-impermeable deflector 404 and a blood-impermeable elongate element 402 having a plurality of blood-permeable windows 426 that are not permeable to embolic debris. In this embodiment, a distal end 420 of deflector 404 again has a cross-sectional surface area substantially equal to that of ascending aorta 422 where deflector 404 is deployed, thereby directing all blood flow into deflector 404. In this embodiment however, deflector 404 is blood-impermeable and perfusion to left subclavian artery 414, left common carotid artery 416 and brachiocephalic artery 418 is made possible by retrograde flow through one or more blood-permeable windows 426. Likewise, perfusion through aortic arch 412 to descending aorta 424 and downstream vasculature is also made possible by flow through one or more blood-permeable windows 426. In this embodiment, due to the generally frustoconical shape of deflector 404, no contact is made with any of the openings to left subclavian artery 414, left common carotid artery 416 and brachiocephalic artery 418. Also illustrated in FIG. 4A is a one-way valve 427 positioned on the outer surface of one of blood-permeable windows 426 so as to substantially cover such window. One-way valve 427 is further comprised of a single slit so as to bias one-way valve 427 to open in one direction. In this manner, one-way valve 427 can be configured to permit perfusion therethrough yet prevent blood from entering, for example, during an aspiration procedure or in diastole.

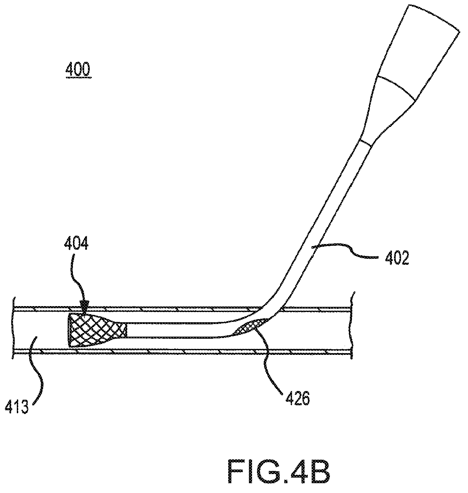

Somewhat similar to FIG. 4A, FIG. 4B illustrates an embolic protection device 400 deployed in a carotid artery 413 comprising a blood-impermeable deflector 404 and a blood-impermeable elongate element 402 having a blood-permeable window 426 that is not permeable to embolic debris. In this embodiment, thoracic interventions through carotid access can be performed while providing cranial perfusion and filtering blood of embolic debris. It should be noted that blood-impermeable deflector 404 can be in the form of a compliant balloon. After insertion and appropriate placement, the balloon can be inflated to cause the majority of blood flow to enter the elongate element 402, where it may be subsequently filtered and returned to the downstream vasculature.

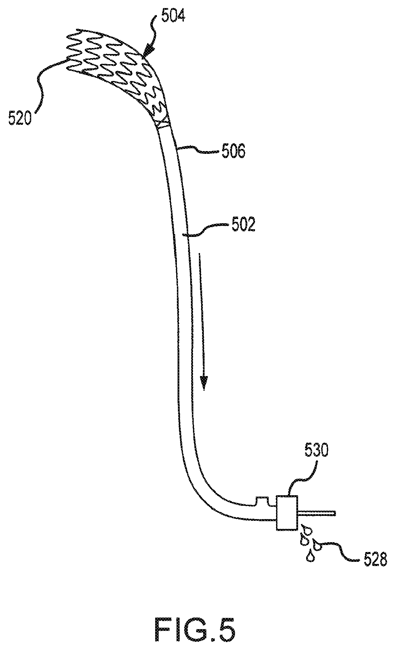

Systems comprising embolic protection devices are also disclosed. With reference to FIG. 5, a system can comprise an elongate element 502 having a deflector 504 at the distal end 506 of elongate element 502. A distal end 520 of deflector 504 can be configured to receive blood and redirect and/or funnel embolic debris 528 through the lumen of deflector 504 and elongate element 502 to be aspirated at a stopcock 530 outside of the patient's body.

Embodiments of methods are also disclosed. In accordance with an embodiment, the method comprises delivering an embolic protection device to a treatment site and removing a sheath from a deflector of the embolic protection device, wherein the sheath is restraining the deflector in a radially collapsed delivery configuration. The deflector can be self-expandable (e.g., comprise a shape-memory support frame, such as nitinol) or otherwise expandable (e.g., with a fluid-filled balloon). In either embodiment, the deployed deflector then has a radially expanded funneling configuration.

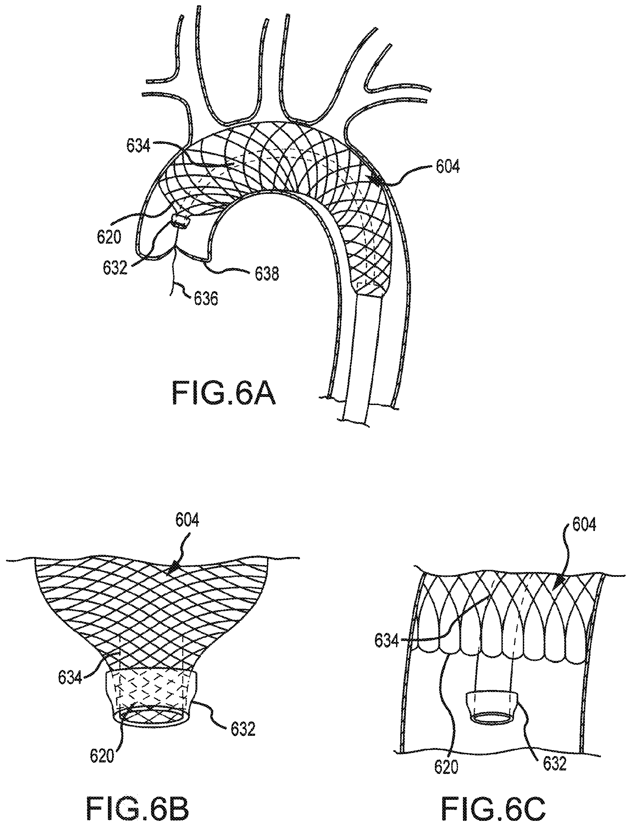

In embodiments, and with reference to FIGS. 6A-6C, a distal end 620 of a deflector 604 can be restrained separately in a radially collapsed configuration after a first stage deployment of deflector 604. For instance, a distal end 620 of a deflector 604 can be restrained separately by a skirt 632 attached to the distal end of a guide element 634, as illustrated in FIGS. 6A-6B, the latter being a close-up of the former. Guide element 634 may be a guide catheter or other elongate element. In such embodiments, deflector 604 can be configured to allow perfusion therethrough even while the distal end of deflector 604 is in a radially collapsed configuration after a first stage deployment of deflector 604. In a second stage deployment of deflector 604, distal end 620 can be freed from skirt 632, for instance by advancing guide element 634 as illustrated in FIG. 6C. These embodiments can provide a "centering" effect, for example, to facilitate crossing of a guide wire 636 (illustrated in FIG. 6A) or one or more secondary endovascular devices or prostheses through an aortic valve 638. Persons skilled in the art will appreciate that beyond a "skirt," any constraining element can be used to radially restrain a distal end of deflector 604 including, but not limited to "purse strings," clips, bioabsorbable materials, etc.

A method further comprises performing all necessary procedures (which can in turn involve inserting one or more secondary endovascular devices through a working lumen of an elongate element of the embolic protection device) and aspirating embolic debris redirected and/or funneled by the embolic protection device as necessary. Finally, one or more secondary endovascular devices can be removed after which the embolic protection device itself can be removed.

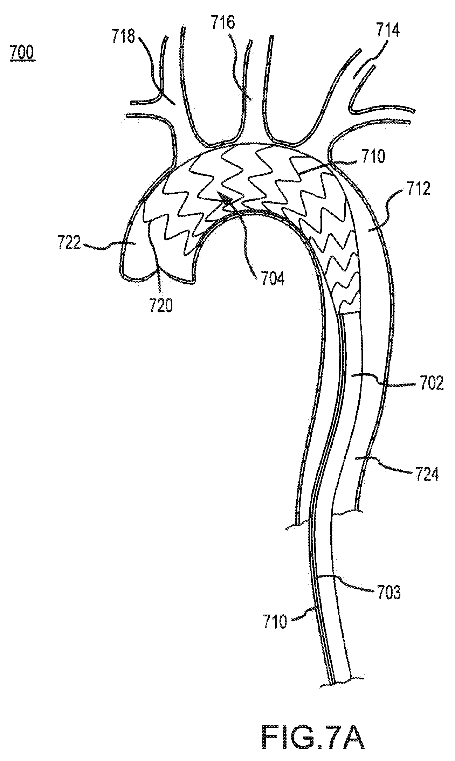

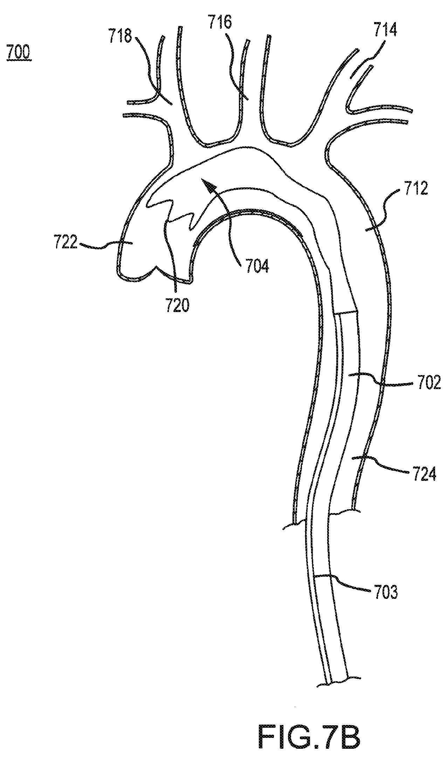

In embodiments, a deflector can be deconstructed in situ by applying tension to a structural support element. In this regard, and with reference to FIG. 7A, an embolic protection device 700 is illustrated deployed in aortic arch 712 spanning left subclavian artery 714, left common carotid artery 716 and brachiocephalic artery 718 and having a deflector 704 and an elongate element 702 extending into descending aorta 724. Elongate element 702 comprises a separate lumen 703 through which passes a proximal portion of, or a line attached to, a deflector structural support element 710. In this manner, deflector 704 can be deconstructed in situ by applying tension to deflector structural support element 710 through separate lumen 703. In the embodiment illustrated in FIG. 7B, deconstruction comprises removal of deflector structural support element 710 from the material of deflector 704, rendering deflector 704, including a distal end 720 of deflector 704, "flimsy" and otherwise having a lower crossing profile and being suitable for removal from the treatment site. As discussed supra, deconstruction can comprise removal of a structural support element together with the material of a deflector, however, removal from the material of a deflector may be advantageous in instances when some residual embolic debris may not have passed into an elongate element. In embodiments, deflector 704 can finally be withdrawn from ascending aorta 722 and descending aorta 724, for example, by recapturing with an over-sheath.

By way of non-limiting example, a method for embolic protection in the aortic arch comprises providing a device comprised of (i) an elongate element having a proximal opening, a distal opening, and a lumen extending therethrough; (ii) a deflector at the distal opening of the elongate element configured to redirect embolic debris into the lumen; and (iii) a guidewire. A method also comprises advancing the device to the aortic arch of a patient's vasculature, followed by deploying a proximal portion of the deflector, wherein a distal end of the deflector is positioned between the aortic valve and the brachiocephalic artery, and wherein the distal end of the deflector is radially restrained. A method further comprises advancing the guidewire across the aortic valve. A method also comprises releasing the distal end of the deflector, followed by performing a therapeutic interventional procedure. A method further comprises aspirating embolic debris from the proximal opening of the elongate element. A method also comprises partially withdrawing the device from the patient's vasculature, wherein the distal end of the deflector is positioned proximal to the left subclavian artery, followed by deconstructing the deflector in situ by applying tension to a structural support element. A method finally comprises completely withdrawing the device from the patient's vasculature.

### Example 1

Embodiments of methods of making embolic protection devices are also provided. In an embodiment, a deflector comprises a conical embolic filter net and a deflector structural support element. The conical embolic filter net is fabricated from an appropriately sized conical shaped filter element from a sintered ePTFE film with approximately 100 micron diameter holes (or smaller, depending on the particular application) laser drilled through the wall. An appropriately shaped conical mandrel may be used.

In an embodiment, a deflector structural support element comprises a self-expanding nitinol frame and is fabricated from a sinusoidal, helical nitinol wire from.010 diameter wire. An appropriately shaped winding jig may be used. The nitinol frame is heat set at approximately 450C for approximately 20 minutes.

Next, the conical embolic filter net and the nitinol frame are assembled. An FEP powder coat is applied to the nitinol frame, after which it is heat treated at approximately 320C for approximately 2 minutes. The powder-coated nitinol frame is placed on an appropriately configured mandrel. Over the nitinol frame is placed the conical embolic filter net. The assembly is overwrapped with a sacrificial layer of ePTFE to apply compression, after which it is heat treated at approximately 320C for approximately 15 minutes. The assembly is air cooled and removed from the mandrel.

Finally, the sub-assembly above (comprising the conical embolic filter net and the nitinol frame) and the introducer tip are assembled. The small diameter portion of the sub-assembly is coupled to an appropriately sized introducer sheath using a swaged radiopaque marker band and a biocompatible adhesive. Methods can be scalable depending on the particular application.

It will be apparent to those skilled in the art that various modifications and variations can be made in the present disclosure without departing from the scope of the disclosure. For example, while embodiments of the present disclosure have been described primarily with reference to the aortic arch, embodiments are scaleable and applications in various peripheral vessels and lumens are contemplated herein. Likewise, as it relates to the aortic arch, the present disclosure can be used in connection with femoral, transapical and thoracotomy approaches. Additionally, the embodiments can be used in connection with not just humans, but also various organisms having mammalian anatomies. Thus, it is intended that the embodiments described herein cover the modifications and variations of this disclosure provided they come within the scope of the appended claims and their equivalents.

Numerous characteristics and advantages have been set forth in the preceding description, including various alternatives together with details of the structure and function of the devices and/or methods. The disclosure is intended as illustrative only and as such is not intended to be exhaustive. It will be evident to those skilled in the art that various modifications can be made, especially in matters of structure, materials, elements, components, shape, size and arrangement of parts including combinations within the principles of the invention, to the full extent indicated by the broad, general meaning of the terms in which the appended claims are expressed. To the extent that these various modifications do not depart from the scope of the appended claims, they are intended to be encompassed therein.

## Claims

1. An embolic protection device comprising:
an elongate element (224) having a proximal end, a distal end, and a lumen extending therethrough; and
a deflector (204) at the distal opening of the elongate element,
wherein the elongate element (224) and the deflector (204) are impermeable to embolic debris greater than about 100µm;
wherein the deflector (204) is blood- impermeable and the elongate element (224) is blood-permeable; and
wherein the elongate element (224)is configured to permit antegrade and retrograde perfusion of blood therethrough.

2. The embolic protection device of claim 1, wherein the deflector (204) is configured to redirect and/or funnel embolic debris greater than about 100 µm into the elongate element (224).

3. The embolic protection device of claim 1, wherein the deflector (204) is configured to not capture or retain embolic debris.

4. The embolic protection device of claim 1, wherein the deflector (204) has a generally frustoconical and/or cylindrical shape.

5. The embolic protection device of claim 4, wherein the deflector (604) is selectively diametrically adjustable.

6. The embolic protection device of claim 1, wherein the deflector (204) comprises a radiopaque element.

7. The embolic protection device of claim 1, wherein the elongate element (402) comprises a plurality of blood-permeable windows (426).

8. The embolic protection device of claim 1, wherein the elongate element (224) comprises a separate lumen for delivering a blood contrast agent.

9. The embolic protection device of claim 1, wherein the deflector (204) comprises a therapeutic agent.

10. The embolic protection device of claim 9, wherein the therapeutic agent comprises heparin.

11. The embolic protection device of claim 1, wherein the elongate element (224) comprises a tension line to cause bending of the elongate element.

12. The embolic protection device of claim 11, wherein bending of the elongate element (224) is controlled remotely by an operator thereby reducing the dependence on the vessel wall for alignment.

13. The embolic protection device of claim 7, wherein at least one of the blood permeable windows (426) further comprises a one-way valve (427) configured to permit perfusion external to the elongate element.

14. The embolic protection device of claim 1 wherein the deflector (604) or a portion thereof is restrained in a radially collapsed delivery configuration by a constraining element (632), optionally wherein a distal end of the deflector (604) is maintained separately in the radially collapsed configuration after deployment of a proximal portion of the deflector (604).

15. The embolic protection device of claim 14 wherein the constraining element (632) is attached to the distal end of a guide element (634), and wherein the distal end of the deflector (604) can be freed from the constraining element (632) by advancing the guide element (634).

## Patentansprüche

1. Embolieschutzvorrichtung, umfassend:
ein elongiertes Element (224) mit einem proximalen Ende, einem distalen Ende und einem sich dort hindurch erstreckenden Lumen; und
einen Deflektor (204) an der distalen Öffnung des elongierten Elements;
wobei das elongierte Element (224) und der Deflektor (204) für Embolierückstände, die größer sind als etwa 100 µm, undurchlässig sind;
wobei der Deflektor (204) blutundurchlässig ist, und wobei das elongierte Element (224) blutdurchlässig ist; und
wobei das elongierte Element (224) so konfiguriert ist, dass es eine antegrade und retrograde Perfusion von Blut dort hindurch zulässt.

2. Embolieschutzvorrichtung nach Anspruch 1, wobei der Deflektor (204) so konfiguriert ist, dass er Embolierückstände, die größer sind als etwa 100 µm, in das elongierte Element (224) umleitet und/oder schleust.

3. Embolieschutzvorrichtung nach Anspruch 1, wobei der Deflektor (204) so konfiguriert ist, dass er Embolierückstände nicht erfasst oder zurückhält.

4. Embolieschutzvorrichtung nach Anspruch 1, wobei der Deflektor (204) eine allgemein kegelstumpfartige und/oder zylindrische Form aufweist.

5. Embolieschutzvorrichtung nach Anspruch 1, wobei der Deflektor (204) selektiv diametral verstellbar ist.

6. Embolieschutzvorrichtung nach Anspruch 1, wobei der Deflektor (204) ein röntgendichtes Element umfasst.

7. Embolieschutzvorrichtung nach Anspruch 1, wobei das elongierte Element (402) eine Mehrzahl blutdurchlässiger Fenster (426) umfasst.

8. Embolieschutzvorrichtung nach Anspruch 1, wobei das elongierte Element (402) ein separates Lumen für die Zufuhr eines Blutkontrastmittels umfasst.

9. Embolieschutzvorrichtung nach Anspruch 1, wobei der Deflektor (204) ein Therapeutikum umfasst.

10. Embolieschutzvorrichtung nach Anspruch 9, wobei das Therapeutikum Heparin umfasst.

11. Embolieschutzvorrichtung nach Anspruch 1, wobei das elongierte Element (224) eine Spannungsleitung umfasst, um eine Biegung des elongierten Elements zu bewirken.

12. Embolieschutzvorrichtung nach Anspruch 11, wobei die Biegung des elongierten Elements (224) aus der Ferne durch eine Bedienungsperson geregelt wird, wodurch die Abhängigkeit von der Gefäßwand für die Ausrichtung verringert wird.

13. Embolieschutzvorrichtung nach Anspruch 7, wobei wenigstens eines der blutdurchlässigen Fenster (426) ferner ein Einwegventil (427) umfasst, das so konfiguriert ist, dass es eine externe Perfusion zu dem elongierten Element ermöglicht.

14. Embolieschutzvorrichtung nach Anspruch 1, wobei der Deflektor (604) oder ein Teil dessen durch ein Einschränkungselement (632) in einer radial kollabierten Zufuhrkonfiguration zurückgehalten wird, wobei optional ein distales Ende des Deflektors (604) nach dem Einsatz eines proximalen Teils des Deflektors (604) separat in der radial kollabierten Konfiguration gehalten wird.

15. Embolieschutzvorrichtung nach Anspruch 14, wobei das Einschränkungselement (632) an dem distalen Ende eines Führungselements (634) angebracht ist, und wobei das distale Ende des Deflektors (604) durch Vorschub des Führungselements von dem Einschränkungselement (632) gelöst werden kann.

## Revendications

1. Dispositif de protection embolique comprenant :
un élément allongé (224) ayant une extrémité proximale, une extrémité distale et une lumière s'étend au travers ; et
un déflecteur (204) au niveau de l'ouverture distale de l'élément allongé,
l'élément allongé (224) et le déflecteur (204) étant imperméables aux débris emboliques supérieurs à environ 100 µm ;
le déflecteur (204) étant imperméable au sang et l'élément allongé (224) étant perméable au sang ; et
l'élément allongé (224) étant conçu pour permettre la perfusion antérograde et rétrograde de sang au travers.

2. Dispositif de protection embolique selon la revendication 1, le déflecteur (204) étant conçu pour rediriger et/ou canaliser les débris emboliques supérieurs à environ 100 µm dans l'élément allongé (224).

3. Dispositif de protection embolique selon la revendication 1, le déflecteur (204) étant conçu pour ne pas capturer ou retenir les débris emboliques.

4. Dispositif de protection embolique selon la revendication 1, le déflecteur (204) ayant une forme généralement tronconique et/ou cylindrique.

5. Dispositif de protection embolique selon la revendication 4, le déflecteur (604) étant sélectivement diamétralement réglable.

6. Dispositif de protection embolique selon la revendication 1, le déflecteur (204) comprenant un élément radio-opaque.

7. Dispositif de protection embolique selon la revendication 1, l'élément allongé (402) comprenant une pluralité de fenêtres (426) perméables au sang.

8. Dispositif de protection embolique selon la revendication 1, l'élément allongé (224) comprenant une lumière séparée pour administrer un produit de contraste du sang.

9. Dispositif de protection embolique selon la revendication 1, le déflecteur (204) comprenant un agent thérapeutique.

10. Dispositif de protection embolique selon la revendication 9, l'agent thérapeutique comprenant l'héparine.

11. Dispositif de protection embolique selon la revendication 1, l'élément allongé (224) comprenant une ligne de tension pour provoquer la flexion de l'élément allongé.

12. Dispositif de protection embolique selon la revendication 11, la flexion de l'élément allongé (224) étant commandée à distance par un opérateur, réduisant ainsi la dépendance envers la paroi des vaisseaux pour l'alignement.

13. Dispositif de protection embolique selon la revendication 7, au moins l'une des fenêtres (426) perméables au sang comprenant en outre une valve unidirectionnelle (427) conçue pour permettre une perfusion externe à l'élément allongé.

14. Dispositif de protection embolique selon la revendication 1, le déflecteur (604) ou une partie de celui-ci étant retenu dans une configuration d'administration radialement effondrée par un élément de contrainte (632), éventuellement une extrémité distale du déflecteur (604) étant maintenue séparément dans la configuration radialement effondrée après le déploiement d'une partie proximale du déflecteur (604).

15. Dispositif de protection embolique selon la revendication 14, l'élément de contrainte (632) étant fixé à l'extrémité distale d'un élément de guidage (634), et l'extrémité distale du déflecteur (604) pouvant être libérée de l'élément de contrainte (632) en avançant l'élément de guidage (634).
